# EUROPEAN PATENT APPLICATION

(11) **EP 0 987 331 A1**
(43) Date of publication of application: **22.03.2000**
(21) Application number: 98116493.2
(22) Date of filing: 01.09.1998
(51) Int. Cl.: C12N 15/82, C12N 9/14

(54) **Plant pathogenicity control by use of a pathogen inducible expression of deac gene**

(71) Applicant: Hoechst Schering AgrEvo GmbH, 13509 Berlin (DE)
(72) Inventor: Bartsch, Klaus Dr., 61462 Königstein (DE)

(57) **Abstract**

Instant invention relates to a method of controlling plant pathogens by pathogen inducible expression of a deacetylase gene (deac) encoding a protein exhibiting N-acetyl-PPT (e.g., N-acetyl-phosphinothricine) specific deacetylase activity in combination with the application of a composition containing one or more non-toxic compound(s) (e.g., N-acetyl-PPT), allowing - after deacetylation within the plant cell - inducible and selective destruction of cells within a living plant after pathogen infestation.

## Description

Instant invention relates to a method of controlling plant pathogens by pathogen inducible expression of a deacetylase gene (deac) encoding a protein exhibiting N-acetyl-PPT (e.g., N-acetyl-phosphinothricine) specific deacetylase activity (DEAC) in combination with the application of a composition containing one or more non-toxic compound(s) (e.g., N-acetyl-PPT), allowing - after deacetylation within the plant cell - inducible and selective destruction of cells within a living plant after pathogen infestation.

One of the main problems in plant protection is the control of plant pathogens. There are many different methods known in the art in order to control plant-pathogens such as viruses, fungi, bacteria, insects, acarina, nematodes, gastropoda or others. Those methods are mainly based on the treatment of the plants, seeds or the area under cultivation with compositions comprising pathogen-toxic chemicals or biologicals.

General problems associated with the treatment of plants with, e.g., chemical insecticides are the development of resistances, the persistence of the chemicals in the environment, and undesired side-effects on non-target organisms.

Another technical disadvantage during application of a pathogen-directed toxin is the appropriate time frame of application, since effective control may be only achieved within a specific developmental stage of the pathogen.

These problems are addressed by the deac system according to instant invention which provides an advantageous solution by inducing a hypersensitive response upon chemical treatment.

Various approaches have been undertaken to produce pathogen-resistant transgenic plants, which are sensitized to respond more rapidly to infection and thus are protected against virulent pathogens. One possible way is to express a pathogen toxine under control of a constitutive promoter in order to control pathogen attack e.g., crystal protein derived from *Bacillus thuringiensis* (US 5,322,938), insect-specific peptidase inhibitors (WO 95/35031), sarcotoxin (EP-A-0 798 381), ribozymes (US 5,641,673), chitinase (US 4,940,840) etc..

The expression of a pathogen-directed toxin within a plant cell, however, does not always provide a solution to the problem of pathogen attack, due to enforcing development of resistances.

A different approach is the expression of a pathogen toxine under control of a pathogen inducible promoter to induce a hypersensitive response, for example, the barnase system (cf. US 5,750,386).

It is a known problem that many of tissue- or development-specific plant promoters are "leaky", i.e., they are also expressed to a certain extent in other tissues or developmental stages or are also inducible by other environmental stress factors than pathogen infection. Thus, the transgenic plants may suffer from cell damage caused by undesired expression of the toxins and result in a loss in productivity.

Therefore, it is an object of the present invention to provide a solution for effective pathogen control in plants while avoiding resistance development and other undesired side effects.

This object has been achieved by use of the deac system according to the invention and by the provision of the embodiments described herein.

The use of the deac system for the selective ablation of plant tissue and cells is known from EP-A-0 531 716. Depending on the type of promoter used, specific cell ablation can be achieved for any plant tissue of interest, e.g., for the induction of transgenic male or female sterility by anther or pistil specific expression of a deacetylase gene. By fusing a deac gene with a tapetum-specific promoter male sterility (MS) can be induced by external application of N-acetyl-phosphinothricine. The N-acetyl-phosphinothricine-specific deacetylase protein (DEAC), the expression product of the deac gene, converts the non-toxic N-acetyl-derivative of phosphinothricine into the herbicidal active compound (WO 98/27201).

Genes coding for suitable deacetylase proteins (DEAC) with appropriate substrate specificities have been described previously: *argE* from *Esocherichia coli*, encoding N-acetyl-ornithine deacetylase, *dea* from *Streptomyces viridochromogenes* (EP-A-0 531 716), *deac1* from *Stenotrophomonas sp.* (DSM 9734) and *deac2* from *Comamonas acidovorans* (DSM 11070), both encoding hippurate hydrolases (WO 98/27201).

Now, it has been surprisingly found that the use of the deac system, i.e. the expression of a deac gene within a plant tissue under control of a pathogen inducible promoter, optionally within a specific tissue (e.g., roots, leaves, fruits, seeds) in combination with the external application of N-acetyl PPT is suitable for pathogen control by selective destruction of the infested plant cells or tissues.

Surprisingly, when using the deac system according to the invention, the formation of the plant-toxic compound (i.e. deacetylated PPT) in the area of penetration is fast enough to trigger a "hypersensitive" response causing rapid cell death and therefore, prevents the pathogen further spreading throughout the plant or plant tissue.

Furthermore, the use of the deac system for pathogen control by selective ablation of pathogen infested plant tissue and cells has a number of advantages, e.g., with respect to resistance development or the broad effectivity against plant pathogens, compared to already known sytems for plant pathogen control.

Using the deac system according to the invention cell death can only be induced by a combination of deac expression and application of N-acetyl PPT. The DEAC activity as such is not harmful and thus, plant cells are not affected by deac expression in case of use of a "leaky" promoter.

Another advantage of the deac system according to the invention is that the optimal time window for the application of N-acetyl-PPT, resp., PPT can be chosen by the farmer depending on pathogen attack.

Therefore, in a first aspect the present invention relates to a nucleic acid molecule comprising the coding region of, resp., a nucleotide encoding a N-acetyl-PPT specific deacetylase under control of a pathogen inducible regulatory element, such as a promoter or a promoter in combination with an enhancer.

Additionally, an object of the invention is the use of a deac gene under control of a pathogen inducible regulatory element, preferably a promoter, or a nucleic acid molecule of the invention for the preparation of a pathogen-tolerant plant cell or plant.

The nucleic acid molecule according to the invention is preferably a DNA or RNA molecule, for instance genomic or cDNA. The nucleotides coding for a N-acetyl-PPT specific deacetylase and/or a pathogen inducible promoter, which are part of the nucleic acid molecule of the invention, may be isolated from natural sources or synthesized by already known methods (Sambrook et al., Molecular Cloning, A Laboratory Manual, 2. Aufl. (1989) Cold Spring Harbor Laboratory Press, Cold Spring Harbor, NY).

The nucleic acid molecule according to the invention may also comprise a fragment, derivative or allelic variant of said nucleotide coding for a N-acetyl-PPT specific deacetylase provided that the biological activity of a N-acetyl-PPT specific deacetylase is restored.

Further, the nucleic acid molecule according to the invention may also comprise fragments, derivatives and allelic variants of a pathogen inducible promoter provided that the biological activity of a pathogen inducible promoter is restored.

The fragments, derivatives and allelic variants of said nucleotides coding for a N-acetyl-PPT specific deacetylase or a pathogen inducible promoter might have been caused by one or more mutations such as deletions, substitutions, insertions and/or recombinations, which may have naturally occured, or which may have been induced by genetic engineering or molecular biological techniques (e.g., Sambrook et al., loc.cit.).

In another embodiment the nucleic acid molecule according to invention is linked to one or more regulatory elements, which ensure transcription and synthesis of a translatable RNA in procaryotic or eucaryotic cells (e.g., enhancer, tissue-specific elements, 3'-poly A-tail, e.g., Gielen et al., EMBO J. 1989, 8:23-29).

When expressing a nucleic acid molecule according to the invention in a plant cell, in general, the expression product, i.e. the deacetylase protein, can be localized in any compartment (e.g., cytosol, vacuole, apoplast, cell wall, plastid, mitochondrium etc.) of the transformed plant cell. In order to target the expression product into a specific compartment, it is required to add to the coding sequence of said nucleic acid molecule a nucleotide coding for a signal peptide, resp., targeting sequence. Such a nucleotide coding for a signal peptide, resp., targeting sequence is well known in the art (e.g., Braun at al., EMBO J. 11 (1992), 3219-3227; Wolter et al., Proc. Natl. Acad. Sci. USA 85 (1988), 846-850; Sonnewald at al., Plant J. 1 (1991), 95-106).

Furthermore, the invention relates to vectors, preferably plasmids, cosmids, viruses, bacteriophages, and other vectors common in genetic engeneering, which comprise a nucleic acid molecule according to invention.

In a further embodiment the invention relates to a host cell, preferably procaryotic or eucaryotic cell, comprising a nucleic acid molecule or a vector according to the invention and the progeny of said host cell.

The host cell of the invention is characterized in that it has been transformed and/or genetically modified by a nucleic acid molecule according to the invention or by a vector according to the invention. The progeny of said host cell is further characterized by the presence of the nucleic acid molecule according to the invention. Preferably, the host cell is a bacterial cell. The host cell of the invention is further characterized in that the introduced nucleic acid molecule is of heterologous origin, i.e. it does not occur naturally in these cells.

Moreover, the present invention relates to a process for the production of a transgenic plant cell comprising the step of introducing a nucleic acid molecule or a vector according to the invention into the genome of a plant cell by known transformation technologies, e.g., by stable integration into the genome of said plant cell, said transgenic plant cell expressing a N-acetyl-PPT specific deacetylase under control of a pathogen inducible regulatory element.

The transformed plant cell obtainable by the process of the invention is a further embodiment of the invention. The plant cell according to the invention expresses a N-acetyl-PPT specific deacetylase under control of a pathogen inducible promoter and is further characterized in that the introduced nucleic acid molecule is of heterologous origin, i.e. it does not occur naturally in these cells.

In another embodiment of the invention the transformed plant cell of the invention additionally expresses a nucleic acid molecule conferring PPT-resistance as known, e.g., by EP-A-0 242 236, EP-A-0 242 246, EP-A-0 257 542 or EP-A-0 531 716, obtainable, e.g., by introducing a known PPT-resistant or tolerant plant cell into the process according to the invention..

By means of methods known to the skilled person in the art the transgenic plant cell of the invention can be regenerated to a complete plant.

Therefore, a further object of the invention is a process for the production of a transgenic plant comprising the step of regenerating a complete plant from a plant cell according to invention, said plant exhibiting pathogen-resistance after treatment with N-acteyl-PPT. Such a plant is obtainable by introducing a plant cell of the invention into a well known process for plant regeneration. If a plant cell comprising a gene conferring PPT-resistance (e.g., a pat or bar gene) is transformed with a nucleic acid molecule or a vector according to the invention, such a plant exhibits pathogen-resistance after treatment with either N-acetyl-PPT and/or PPT.

Accordingly, if a plant according to the invention exhibits additionally PPT-tolerance, the treatment of said plant with PPT results simultaneously in a herbicidal and pathogen-controlling effect.

In order to prepare the integration of foreign nucleic acid molecules into higher plants a high number of cloning vectors are available, containing a replication signal for E.coli and a marker gene for the selection of transformed bacterial cells. Examples of such vectors are pBR322, pUC series, M13mp series, pACYC184, pBinAR etc. The desired sequence may be integrated into the vector at a suitable restriction site. The obtained plasmid is used for the transformation of E.coli cells. Transformed E.coli cells are cultivated in a suitable medium and subesequently harvested and lysed. The plasmid is recovered. As an analyzing method for the characterization of the obtained plasmid DNA, use is generally made of restriction analysis, gel electrophoresis and other biochemical or molecularbiological methods. After each manipulation the plasmid DNA may be cleaved and the obtained DNA fragments may be linked to other DNA sequences. Each plasmid DNA may be cloned into the same or other plasmids. In order to integrate DNA into plant host cells, a wide range of techniques are available. These techniques comprise the transformation of plant cells with T-DNA by using Agrobacterium tumefaciens or Agrobacterium rhizogenes as transformation medium, by polyethylene glycol treatment, the fusion of protoplasts, the injection or electroporation of DNA, the integration of DNA by means of the biolistic method as well as further possibilities.

In the case of injection and electroporation of DNA into plant cells, there are no special demands made on the plasmids used. Simple plasmids such as pUC derivatives may be used. However, in case that whole plants are to be regenerated from cells transformed in such a way, a selectable marker gene should be present.

Depending on the method of integrating desired genes into the plant cell, further DNA sequences may be necessary. If the Ti- or Ri-plasmid is used e.g. for the transformation of the plant cell, usually at least the right border, more frequently, however, the right and left border of the Ti- and Ri-plasmid T-DNA should be connected to the foreign gene to be integrated as a flanking region.

If Agrobacteria are used for the transformation, the DNA which is to be integrated should be cloned into special plasmids, namely either into an intermediate vector or into a binary vector. Due to sequences homologous to the sequences within the T-DNA, the intermediate vectors may be integrated into the Ti- or Ri-plasmid of the Agrobacterium due to homologous recombination. This also contains the vir-region necessary for the transfer of the T-DNA. Intermediate vectors cannot replicate in Agrobacteria. By means of a helper plasmid the intermediate vector may be transferred to Agrobacterium tumefaciens (conjugation). Binary vectors may replicate in E.coli as well as in Agrobacteria. They contain a selectable marker gene as well as a linker or polylinker which is framed by the right and the left T-DNA border region. They may be transformed directly into Agrobacteria (Holsters et al. Mol. Gen. Genet. 163 (1978), 181-187). The Agrobacterium acting as host cell should contain a plasmid carrying a vir-region. The vir-region is usually necessary for the transfer of the T-DNA into the plant cell. Additional T-DNA may be present. The Agrobacterium transformed in such a way is used for the transformation of plant cells.

The use of T-DNA for the transformation of plant cells was investigated intensively and described sufficiently in EP 120 516; Hoekema, In: The Binary Plant Vector System Offsetdrukkerij Kanters B.V., Alblasserdam (1985), Chapter V; Fraley et al., Crit. Rev. Plant. Sci., 4, 1-46 and An et al. EMBO J. 4 (1985), 277-287.

For transferring the DNA into the plant cells, plant explants may suitably be co-cultivated with Agrobacterium tumefaciens or Agrobacterium rhizogenes. From the infected plant material (e.g. pieces of leaves, stem segments, roots, but also protoplasts or suspension-cultivated plant cells) whole plants may then be regenerated in a suitable medium which may contain antibiotics or biocides for the selection of transformed cells. The plants obtained in such a way may then be examined as to whether the integrated DNA is present or not. Other possibilities in order to integrate foreign DNA by using the biolistic method or by transforming protoplasts are known to the skilled person (cf. e.g. Willmitzer, L., 1993 Transgenic plants. In: Biotechnology, A Multi-Volume Comprehensive Treatise (H.J. Rehm, G. Reed, A. Pühler, P. Stadler, editors), Vol. 2, 627-659, VCH Weinheim-New York-Basel-Cambridge).

Alternative systems for the transformation of monocotyledonous plants are transformation by means of a biolistic approach, the electrically or chemically induced DNA integration in protoplasts, the electroporation of partially permeabilized cells, the macro-injection of DNA into inflorescences, the micro-injection of DNA into microspores and pro-embryos, the DNA integration by sprouting pollen and the DNA integration in embryos by swelling (review given in: Potrykus, Physiol. Plant (1990), 269-273).

Whereas the transformation of dicotyledonous plants by Ti-plasmid-vector systems by means of Agrobacterium tumefaciens is a well-established method, more recent studies indicate that the transformation with vectors based on Agrobacterium can also be used in the case of monocotyledonous plants (Chan et al., Plant Mol. Biol. 22 (1993), 491-506; Hiei et al., Plant J. 6 (1994), 271-282; Bytebier at al., Proc. Natl. Acad. Sci. USA 84 (1987), 5345-5349; Raineri et al., Bio/Technology 8 (1990), 33-38; Gould at al., Plant Physiol. 95 (1991), 426-434; Mooney et al., Plant, Cell Tiss. & Org. Cult. 25 (1991), 209-218; Li et al., Plant Mol. Biol. 20 (1992), 1037-1048, Jähne at al., Euphytica 85 (1995), 35-44, Maheshwari et al., Critical Reviews in Plant Science 14 (2) (1995), 149-178, Hess et al. (Plant Sci. 72 (1990), 233, Vasil at al., (Bio/Technology 10 (1992), 667-674, Weeks at al. (Plant Physiol. 102 (1993), 1077-1084, Becker et al. (Plant J. 5 (2) (1994), 299-307.

Once the introduced DNA has been integrated in the genome of the plant cell, it usually continues to be stable there and also remains within the descendants of the originally transformed cell. It usually contains a selectable marker which confers resistance against a biocide such as phosphinotricine or against an antibiotic such as kanamycin, G 418, bleomycin or hygromycin etc. to the transformed plant cells. The individually selected marker should therefore allow for a selection of transformed cells to cells lacking the integrated DNA.

The transformed cells grow in the usual way within the plants (see also McCormick et al., Plant Cell Reports 5 (1986), 81-84). The resulting plants can be cultivated in the usual way and cross-bred with plants having the same transformed genetic heritage or another genetic heritage. The resulting hybrid individuals have the corresponding phenotypic properties. The plant cells produce seeds.

Two or more generations should be grown in order to ensure whether the phenotypic feature is kept stably and whether it is transferred. Furthermore, seeds should be harvested in order to ensure that the corresponding phenotype or other properties will remain.

Another object of the invention is the plant obtainable by a process of the invention, said plant comprising a plant cell of the invention. The plant according to the invention confers pathogen resistance after treatment with N-acetyl PPT and/or PPT. Preferably, the plant of the invention is an angiosperm, gymnosperm, monocotyledonous or dicotyledonous plant, especially a crop plant, in particular a plant for the production of fruits, nuts, vegetables, fodder, cereals, ornamentals, herbs, etc., such as, Dilleniidae, Rosanae, Fabaceae, Anacardiaceae, Euphorbiaceae, Hamamelididae, Solananaceae, Pooideae, especially selected from the group consisting of rye, barley, oat, maize, wheat, millet, cassava, sago, rice, lens, pea, maniok, potato, tobacco, tomato, rape, canola, soya, sugar beet, sugar cane, hemp, cotton, flax, sunflower, mungbean, bean, banana, arrowroot, peanut, broccoli, cucumber, cauliflower, cabbage, lettuce, paprika, apricot, peach, plum, cherry, apple, pear, quince, tea, coffee, orange, lemon, grapefruit, grape, gooseberry, blackberry, cranberry, raspberry, strawberry, blueberry, lupin, clover, vicia faba, chickpea and cashew.

The progeny of said plant or plant cell comprises fruits, seeds, tubers, root-stocks, seedlings, cuttings, calli, cell cultures etc. which are characterized by the presence of the nucleic acid molecule according to the invention.

The hypersensitive reaction induced by the deac system according to the invention does not attack the pathogen directly, but acts by inhibiting further spreading of the infection. Due to this mechanism the deac system according to the invention is not only highly effective against a large number of plant pathogens (viruses, fungi, bacteria, insects, acarina, nematodes and gastropoda), but avoids also the development of resistance phenomenons due to circumvention of the pathogen metabolism. Furthermore, the life cycle of the pathogen is considerably affected resulting in a decreased pathogen attack in the next generation. Therefore, the instant invention provides ecological advantages for man and environment. Furthermore, the invention is not restricted to a single specific pathogen or a specific pathogenic mechanism, but may cover a very broad range or very different pathogens simultaneosly.

Another is a process for controlling one or more pathogens in plants according to the invention, wherein N-acetyl-PPT and/or PPT is applied to said plants or the area under cultivation.

Eventually, it is an object of the invention to use the deac system for pathogen control, and to use N-acetyl-PPT and/or PPT for pathogen control in plants of the invention.

Since the deac system according to the invention offers an optional solution (an option for pathogen control depending on the application of N-acetyl-PPT, resp., PPT), it can be used to optimize agricultural productivity dependent from environmental conditions influenced by pathogen stress etc.. In the case that crop plants are not infested by pathogens, N-acetyl-PPT, resp., PPT need not to be applied.

In this application, the term "deac system" shall have the meaning of one or more gene(s) coding for a protein exhibiting deacetylase activity under control of a pathogen-inducible promoter in combination with the application of one or more non-toxic compounds which act as substrate(s) of said deacetylase(s) or compositions comprising said non-toxic compounds, which - after deacetylation - lead to a limited toxic effect within a plant tissue, plant cell and/or developmental stage of the plant.

The term "deacetylase activity" shall have the meaning of an enzymatic acitivity with a specifity for N-acetylated PPT (N-acetyl-PPT).

The term "PPT" is not restricted to the herbicidal active compound phosphinothricine (2-amino-4-methylphosphinobutyric acid, glufosinate), but shall also comprise derivatives and related compounds such as desmethylphosphinothricine (2-amino-4-hydroxyphosphinobutyric acid), bialaphos (phosphinothricyl-alanyl-alanine), desmethylbialaphos and the like, their salts, racemic mixtures and active enantiomers.

In PPT-tolerant plants, which express, e.g., the phosphinothricin acetyltransferase (*pat* or *bar*) gene, pathogen control or resistance is also achievable by herbicide treatment with PPT. In this case N-acetyl-PPT is generated by the plant itself, and is selectively reconverted to the active herbicide only in the tissue expressing the deac gene.

It is well known in the art, that plants respond to pathogen attack by induced production of defence molecules such as phytoalexins, PR-proteins or protease inhibitors and by a reaction called hypersensitive cell death, which immobilizes the pathogen at the site of penetration. A number of defence genes have been cloned (e.g. pathogen-related (PR)-protein genes, Martins et al., 1993, Mol. Gen. Genet. 236: 179-186) and their pathogen or stress inducible promoters are available (cf. table 1).

In some cases the corresponding promoters have been characterized and pathogen-induced gene expression was demonstrated by GUS-reporter gene fusions.

In this application, the term "pathogen" means any pathogen or pest with which plants can be infested, whether plant- specific or not, such as viruses, bacteria, fungi, insects, acarina, nematodes, gastropoda, and also any other endo- or ectoparasite.

Transgenic plants are generated, expressing a deac gene under control of a pathogen-inducible (e.g., PR-protein or wound-inducible) promoter. The developement of the plants is not affected under normal and under stress conditions, since the deac protein is not toxic to the cells per se. The plants are monitored for pathogen infection and the chemical, e.g., N-acetyl-PPT or PPT is applied just in time before an infection becomes manifest.

**Table 1**

| Examples for pathogen-related or wound-inducible promoters | | | |
|---|---|---|---|
| Promoter | Origin | Pathogen | Reference |
| PRP-1 | *Solanum tuberosum* | *Phytophtora infestans* | Martini et al., (1993), Mol. Gen. Genet. 236:179-183 |
| DRRG49-c | *Phaseolus vulgaris* | *Fusarium solani* | Chiang & Hadwiger, (1990), Mol. Plant Microbe Interact. 3(2):78-85 |
| EAS4 | *Nicotiana tabacum* | *Pseudomonas syringae Phytophtora parasitica* | Yin et al., (1997), Plant Physiol. 115(2):437-451 |
| PR-1a | *Nicotiana tabacum* | Tobacco mosaic virus | Strompen et al., (1998), Plant Mol. Biol. 37:871-883 |
| GN1 | *Nicotiana tabacum* | *Pseudomonas syringae* | Alonso et al., (1995), Plant J. 7(2): 309-320 |
| PR1-1, PR1-2 | *Petroselinum crispum* | *Phytophtora sojae* | Rushton et al., (1996), EMBO J. 15(20):5690-5700 |
| Lemmi9 | *Lycopersicum esculentum* | *Meloidogyne incognita* | van der Eycken et al., (1996), Plant J. 9(1):45-54 |
| mas2' | *Agrobacterium tumefaciens* | wound-inducible | Ni et al., (1996), Plant Mol. Biol. 30 (1): 77-96 |
| tap1 | *Lycopersicum esculentum* | wound-inducible | Mohan et al., (1993), Plant Mol. Biol. 22 (3): 475-490 |
| AoPR1 | *Asparagus officinalis* | wound-inducible | Warner et al., (1993), Plant J. 3 (2): 191-201 |
| wun1 | *Solanum tuberosum* | wound-inducible | Logemann and Schell, (1989), Mol. Gen. Genet. 219 (1-2): 81-88 |
| win3.12 | *Populus trichocarpa* x *Populus deltoides* | wound-inducible | Hollick and Gordon, (1993), Plant Mol. Biol. 22: 561-572 |
| win6 | *Populus trichocarpa* x *Populus deltoides* | wound-inducible | Clarke et al., (1994), Plant Mol. Biol. 25 (5): 799-815 |

Table 1 provides examples for some pathogen- or wound-inducable promoters.

In addition, the specific deac gene expression in combination with application of N-acetyl-PPT may be combined with other transgenic strategies for pathogen resistance (e.g., antifungal protein, ribosomal inhibiting protein, chitinases, glucanases) or other molecularbiological approaches including qualitative and/or quantitative modification/alteration of plant products, e.g., by cross breeding or transformation technology.

### Example 1: Pathogen control by pathogen-induced cell ablation

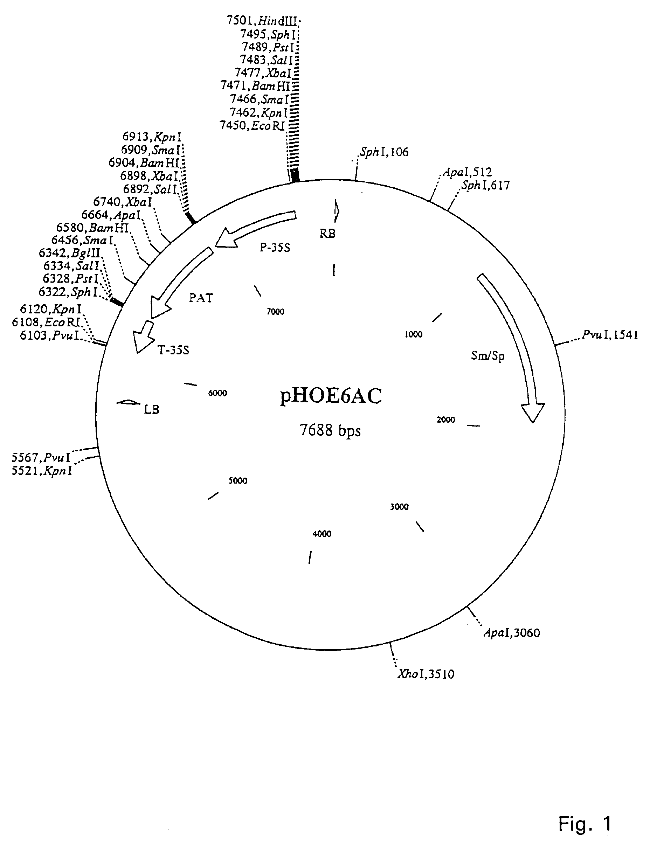

The deac1 structural gene was fused with the pathogen inducible promoter prp1-1 from potato (Martini et al., 1993, Mol. Gen. Genet. 236: 179-186) and the promoter-gene cassette was subcloned in the binary vectors pPCV801 (selectable marker: Pnos-*hpt*, Koncz et al., 1989, Proc. Natl. Acad. Sci. USA 86: 8467-8471) and pHOE6AC (selectable marker: P35S-*pat*, cf. Fig. 1). These constructs were transformed into potato (*Solanum tuberosum*) by *Agrobacterium* mediated gene transfer (de Block, 1988, Theor. Appl. Genet. 76: 767-774).

Transformed plants were selected on hygromycin or phosphinothricin medium, depending on the plasmid construct, and tested for the presence of the transgene by southern blot analysis.

20 plants from each transformation experiment were regenerated and transfered to the green house for further analysis. Since the prp1-1 promoter is known to be inducible by salicylic acid, DEAC activity was measured in crude leaf extracts (WO 98/27201) 2 days after spraying single leaves of the transformants with 1 mg /ml Na salicylate. DEAC-positive plants were further analyzed for sensitivity against N-acetyl-phosphinothricine and phosphinothricine, respectively. Therefore a single leaf of each plant was sprayed with 1 mg/ml Na salicylate as described above. After 2 days 5 µl of N-acetyl-D,L-phosphinothricine dilutions (4 mg/ml , 1 mg/ml, 0.4 mg/ml, 0.1 mg/ml) were spotted on the induced leaves of the pPCV801-transformants. The pHOE6AC-plants were treated in the same manner with dilutions of D,L-phosphinothricine (2 mg/ml, 1mg/ml, 0.5 mg/ml, 0.1 mg/ml). Sensitivity against N-acetyl-phosphinothricine - i.e. necrosis formation - was observed in most of the DEAC-positive plants (pPCV801-transformants) down to 0.4 mg/ml. The effects of phosphinothricine treatment on the pHOE6AC-plants were less pronounced, probably due to the expression of the *pat* gene in those transformants. However, surface irritations and weak necrosis development were observed in many of the deac-positive plants at phosphinothricine concentrations of 2 and 1 mg/ml. Deac transformants without Na salicylate preincubation as well as induced and uninduced wild type and *pat* control plants did not exhibit any tox symptoms after N-acetyl-phosphinothricine and phosphinothricine treatment, respectively.

The transgenic plants with the highest inducible deac expression levels were chosen for infection experiments with *Phytophtora infestans*. Plants were sprayed two-times with 5 mg/ml N-acetyl-D,L-phosphinothricine (pPCV801-transformants) or 2 mg/ml D,L-phosphinothricine (pHOE6AC-transformants) and infected with spore suspension of *Phytophtora infestans* 1 week after the last spraying.

Two of the pPCV801-deac-transformants were fully resistant against the fungal infection. In most of the other pPCV801 -deac plants as well as many of the pHOE6AC-deac-transformants, development and spreading of disease symptoms was significantly delayed compared to infected control plants (deac transformants, unsprayed/ wild type plants sprayed and unsprayed).

## Claims

1. Nucleic acid molecule comprising a nucleotide encoding a N-acetyl-PPT specific deacetylase under control of a pathogen inducible regulatory element.

2. Nucleic acid molecule comprising a nucleotide encoding a N-acetyl-PPT specific deacetylase under control of a pathogen inducible promoter.

3. Nucleic acid molecule according to one or more of claims 1 to 2 which is DNA or RNA.

4. Vector comprising a nucleic acid molecule according to one or more of claims 1 to 3.

5. Host cell comprising a nucleic acid molecule according to one or more of claims 1 to 3 or a vector according to claim 4 and the progeny thereof.

6. A process for the production of a transgenic plant cell comprising the step of introducing a nucleic acid molecule according to one or more of claims 1 to 3 or a vector according to claim 4 into the genome of a plant cell, said transgenic plant cell expressing a N-acetyl-PPT specific deacetylase under control of a pathogen inducible regulatory element.

7. A process according to claim 6 characterized in that the plant cell is PPT-tolerant.

8. Plant cell obtainable by a process according to claim 6 or 7 characterized by expressing a N-acetyl-PPT specific deacetylase under control of a pathogen inducible regulatory element.

9. A process for the production of a transgenic plant comprising the step of regenerating a complete plant from a plant cell according to claim 8.

10. Plant obtainable by the process according to claim 9, characterized in that the plant comprises a plant cell according to claim 8.

11. Plant according to claim 10 which is a crop plant.

12. Progeny of a plant according to one or more of claims 10 to 11.

13. Use of a nucleic acid molecule according to one or more of claims 1 to 3, a vector according to claim 4 or a host cell according to claim 5 for the preparation of a pathogen-tolerant plant cell or plant.

14. Use of the deac gene under control of a pathogen inducible promoter for the preparation of a plant cell or plant.

15. Process for controlling one or more pathogens in plants according to one or more of claims 8 to 10, characterized by application of N-acetyl-PPT and/or PPT to said plants or the area under cultivation.

16. Use of the deac system for pathogen control.

17. Use of N-acetyl-PPT and/or N-acetyl-PPT for pathogen control in a plant according to one or more of claims 10 to 11.
